# EUROPEAN PATENT APPLICATION

(11) **EP 2 050 719 A1**
(43) Date of publication of application: **22.04.2009**
(21) Application number: 07790507.3
(22) Date of filing: 09.07.2007
(51) Int. Cl.: C01G 23/053, A61K 8/27, A61K 8/29, A61Q 17/04, C01G 9/02

(54) **POWDERY TITANIUM OXIDE-ZINC OXIDE AGGREGATE, AND PROCESS FOR PRODUCTION THEREOF**

(30) Priority: 07.07.2006 JP 2006187337
(71) Applicant: Shiseido Company, Limited, Chuo-ku Tokyo 104-8010 (JP)
(72) Inventor: SHIO, Shoichiro, Yokohama-shi Kanagawa 224-8558 (JP); WADA, Masayoshi, Yokohama-shi Kanagawa 224-8558 (JP); SUDA, Yukimitsu, Yokohama-shi Kanagawa 224-8558 (JP); NAGARE, Yuko, Yokohama-shi Kanagawa 224-8558 (JP)
(74) Representative: Henkel, Feiler & Hänzel
(86) International application number: PCT/JP2007/063686
(87) International publication number: WO 2008/004694

(57) **Abstract**

The present invention provides a powder that can display excellent protection ability in both UV-A and UV-B regions. The titanium oxide-zinc oxide aggregate powder comprises an aggregate formed by the aggregation of porous titanium oxide particles and zinc oxide particles, and said porous titanium oxide particle is an aggregate of primary fine particles of titanium oxide. The production method of the titanium oxide-zinc oxide aggregate powder comprises the steps of: (a) hydrolyzing, with heating, an aqueous solution containing a titanium salt and an aliphatic alcohol; (b) adding a water-soluble zinc salt to a suspension of a precipitate obtained by the hydrolysis with heating in step (a), and then neutralizing the suspension with an alkali; and (c) calcining a precipitate obtained by the neutralization in step (b).

## Description

### RELATED APPLICATIONS

This application claims the priority of Japanese Patent Application No. 2006-187337 filed on July 7, 2006, which is incorporated herein by reference.

### FIELD OF THE INVENTION

The present invention relates to a titanium oxide-zinc oxide aggregate powder, and in particular, relates to an aggregate powder that can display a UV protection effect in both UV-A and UV-B regions.

### BACKGROUND OF THE INVENTION

In the field of cosmetics, titanium oxide fine particles and zinc oxide fine particles are used for the purpose of UV protection. Titanium oxide is excellent in mainly UV-B protection ability; however, titanium oxide hardly has UV-A protection ability. On the other hand, zinc oxide is excellent in UV-A protection ability; however, the UV-B protection ability is not as great as that of titanium oxide.

In order to protect against a wide range of ultraviolet light, a combination of zinc oxide and titanium oxide has been tried. However, if zinc oxide and titanium oxide are used together in cosmetics, there is a problem in that heteroaggregation takes place between them and the UV protection ability of both zinc oxide and titanium oxide decreases. Especially, the functional decline of zinc oxide due to heteroaggregation is significant.
In Patent Literature 1 described below, a porous titanium oxide powder, which has a very large specific surface area and is excellent in the UV protection ability, transparency, and usability, is disclosed. However, even such a porous titanium oxide cannot avoid the heteroaggregation with zinc oxide.

Therefore, when zinc oxide and titanium oxide are used together, the mechanical high dispersion treatment in a base is presently carried out with the use of a dispersant. However, a machine for high dispersion treatment is separately necessary, and the process becomes complicated. A surface-treated powder is also widely used for the improvement in dispersion or the suppression of aggregation in cosmetics. However, the surface treatment agent is sometimes removed from the powder by high dispersion treatment.
Thus, a powder that can display excellent UV protection ability in both UV-A and UV-B regions, without high dispersion treatment with a strong mechanical dispersing power, has been hoped for.
Patent Literature 1: Japanese Unexamined Patent Publication No. 2004-292266

### DISCLOSURE OF THE INVENTION

### PROBLEM TO BE SOLVED BY THE INVENTION

The present invention was made in view of the above-described problem of the background art, and the object is to provide a powder that can display excellent protection ability in both UV-A and UV-B regions.

### MEANS TO SOLVE THE PROBLEM

The present inventors have diligently studied to attain the above-described object. As a result, the present inventors found that an aggregate powder of titanium oxide particles and zinc oxide particles could be obtained by a specific method, and this aggregate powder could be excellently dispersed in a base, without high dispersion treatment, and could display high UV protection ability over a wide range from UV-B to UV-A, thus leading to completion of the present invention.
That is, the titanium oxide-zinc oxide aggregate powder of the present invention is characterized by comprising an aggregate formed by the aggregation of porous titanium oxide particles and zinc oxide particles, and wherein said porous titanium oxide particle is an aggregate of primary fine particles of titanium oxide.
In the powder of the present invention, it is preferable that the average particle size of porous titanium oxide particles is 0.01 to 0.5 µm.
It is also preferable that the average particle size of zinc oxide particles is 0.02 to 0.1 µm.
It is also preferable that the average particle size of the aggregate powder is 1 to 300 µm.

The production method of the titanium oxide-zinc oxide aggregate powder of the present invention is characterized by comprising the steps of:
(a) hydrolyzing, with heating, an aqueous solution containing a titanium salt and an aliphatic alcohol;
(b) adding a water-soluble zinc salt to a suspension of a precipitate obtained by the hydrolysis with heating in step (a), and then neutralizing the suspension with an alkali; and
(c) calcining a precipitate obtained by the neutralization in step (b).
   In the method of the present invention, it is preferable that a carboxylic acid is present in step (a) and/or step (b).
   It is also preferable that the precipitate obtained in step (a) is further heat-treated with an acid.
   It is also preferable that the aliphatic alcohol is a polyhydric alcohol.
   It is also preferable that the carboxylic acid is acetic acid.
   It is also preferable that the alkali is sodium carbonate.

### EFFECT OF THE INVENTION

The titanium oxide-zinc oxide aggregate powder obtained in the present invention can be easily dispersed in the base, and display UV protection ability over a wide range from UV-B to UV-A.

### BRIEF DESCRIPTION OF THE DRAWINGS

Fig. 1 is an electron micrograph (SEM image) of the titanium oxide-zinc oxide aggregate powder (Production Example 1) in one example of the present invention.
Fig. 2 shows the transmittance, in the range from 280 nm to 700 nm, for the titanium oxide-zinc oxide aggregate powder (Production Example 1) in one example of the present invention and for a 1:1 mixture of commercial titanium oxide fine particles and commercial zinc oxide fine particles (Comparative Example 1).

### BEST MODE FOR CARRYING OUT THE INVENTION

The aggregate powder of the present invention is formed by the aggregation of mixed porous titanium oxide particles and zinc oxide particles.
Such an aggregate powder can be obtained by:
(a) hydrolyzing, with heating, an aqueous solution containing a titanium salt and an aliphatic alcohol;
(b) adding a water-soluble zinc salt to a suspension of a precipitate obtained by hydrolysis with heating in step (a), and neutralizing the suspension with an alkali; and
(c) calcining a precipitate obtained in the neutralization in step (b).
   Thus, the aggregate powder can be formed by generating zinc oxide in the suspension of titanium oxide.

In the production method of the aggregate powder of the present invention, it is preferable that a carboxylic acid is present in the hydrolysis in step (a).
It is also preferable that a water-soluble zinc salt and a carboxylic acid are present in the suspension to be neutralized with an alkali in step (b).
For example, it is possible that: an aqueous solution containing a titanium salt, aliphatic alcohol, and carboxylic acid is hydrolyzed with heating; to the resulting suspension of precipitate (instead, in the present invention, a suspension obtained by separating the precipitate (hydrolyzate) from the resulting suspension by solid-liquid separation and then redispersing the precipitate into water can be used), a water-soluble zinc salt and a carboxylic acid are added; the suspension is neutralized with an alkali; and then calcination is conducted.
Alternatively, it is possible that: a water-soluble zinc salt and a carboxylic acid are added to an aqueous solution containing a titanium salt and an aliphatic alcohol; the solution is hydrolyzed with heating; the resulting suspension of precipitate is neutralized with an alkali; and then calcination is conducted.

A water-soluble zinc salt and a carboxylic acid can be added to the same system or different systems. For example, it is possible that: a carboxylic acid is added to an aqueous solution containing a titanium salt and an aliphatic alcohol; the solution is hydrolyzed with heating; to the resulting suspension of precipitate, a water-soluble zinc salt is added; the suspension is neutralized by an alkali; and then calcination is carried out. The systems to which an acid and a water-soluble zinc salt are added can be reversed.
Alternatively, it is possible that: a carboxylic acid is added to an aqueous solution containing a titanium salt and an aliphatic alcohol; the solution is hydrolyzed with heating; the resulting precipitate is separated and redispersed into a water; to the obtained suspension, a water-soluble zinc salt and a carboxylic acid are added; the suspension is neutralized; and then calcination is carried out.

In the following, further explanation is provided by listing, as an example, the case in which a water-soluble zinc salt and a carboxylic acid are added after the hydrolysis with heating. However, the present invention is not limited by these examples.

### (a) Hydrolysis with heating

In this step, a titanium salt is hydrolized with heating.
There are no particular limitations on the titanium salt so far as it is a water-soluble titanium salt, and the examples include inorganic titanium salts such as titanium sulfate, titanyl sulfate, and titanium tetrachloride. Among them titanium tetrachloride is preferable.
The concentration of a titanium salt in the aqueous solution, which is subjected to the hydrolysis with heating, can be 0.1 to 5 mol/L. To the aqueous solution of the titanium salt, other water-soluble solvents can be added so far as there are no special effects.

In the hydrolysis with heating, an aliphatic alcohol coexists with a titanium salt. If an aliphatic alcohol is not present, the generated titanium oxide particles would not be porous.
The particle size and specific surface area of the porous titanium oxide particles can be varied by changing the concentration of the aliphatic alcohol. This is considered to be because the aliphatic alcohol affects the particle size and aggregation state of the titanium oxide primary particles; as a result, the particle size and specific surface area of the porous titanium oxide particles, which are the secondary particles, are varied.
The concentration of the aliphatic alcohol is suitably determined in accordance with the purpose; it is normally 0.1 to 5 mol/L in the aqueous solution to be hydrolyzed with heating, and preferably 0.5 to 3 mol/L. If the concentration of the aliphatic alcohol is too low or too high, the particle size and specific surface area of porous titanium oxide particles may be insufficiently.

Examples of aliphatic alcohols are those having 1 to 22 carbon atoms, and include methanol, ethanol, isopropyl alcohol, butyl alcohol, octanol, and stearyl alcohol. The preferable aliphatic alcohol is a polyhydric alcohol.
There are no particular limitations on the polyhydric alcohol, and ethylene glycol, propylene glycol, 1,4-butylene glycol, 2,3-butylene glycol, 1,3-butylene glycol, dimethylpropanediol, diethylpropanediol, glycerol, trimethylolpropane, triethylolpropane, erythritol, xylitol, mannitol, sorbitol, maltitol, etc. can be suitably used.
When a monohydric alcohol is used, the porous titanium oxide particles tend to lose their shape compared with the use of a polyhydric alcohol. An especially preferable aliphatic alcohol is glycerin.

The UV protection effect can be further improved by allowing a carboxylic acid to coexist with a titanium salt and an aliphatic alcohol in the hydrolysis with heating. If a carboxylic acid is allowed to coexist, the carboxyl group coordinates to the precipitate and suppresses the growth of particles; as a result, the particle size suitable to the UV protection effect can be attained.
The concentration of the carboxylic acid is suitably determined in accordance with the purpose; it is normally 0.1 to 5 mol/L in the aqueous solution to be hydrolyzed with heating, and preferably 0.5 to 3 mol/L. If the concentration of the carboxylic acid is too low, the particle size may become too large, resulting in a decrease in the UV protection effect. If the concentration of the carboxylic acid is too high, the particles may become too fine and cause aggregation thereof; as a result, the UV protection effect may become lower.

The conditions for the hydrolysis with heating are suitably determined in accordance with used raw materials and their concentrations; they are normally at 50 to 100 °C for 1 to 12 hours.
After the hydrolysis with heating, the resulting suspension can be used, as it is, for the following step. However, it is preferable to use, for the following step, a suspension that is obtained by separating the precipitate from the resulting suspension by solid-liquid separation and then redispersing the precipitate into water.

As described later, the UV protection effect can also be improved by the heat treatment using an acid after the hydrolysis with heating. If the heat treatment using an acid is carried out, the specific surface area will dramatically become large because of pore enlargement, and the transparency in the visible region and the protection ability in the UV region will become higher. In addition, the particle size of powder tends to become smaller and more uniform, by the heat treatment using an acid, compared with the state before the heat treatment.
The type of acid, concentration thereof, and conditions of heat treatment are suitably determined in accordance with the purpose. Normally, the acid is hydrochloric acid, the concentration is 1 to 8 mole equivalents with respect to titanium, and the conditions of heat treatment are at 50 to 100 °C for 1 to 12 hours.

### (b) Neutralization with Alkali

A water-soluble zinc salt and a carboxylic acid are added to the suspension.
As the water-soluble zinc salt, those normally used in a liquid phase method can be listed; the examples include zinc chloride, zinc nitrate, zinc sulfate, and zinc acetate. Among them, zinc chloride and zinc acetate are preferable, and zinc chloride is especially preferable.
In the suspension to be neutralized with an alkali, the concentration of a water-soluble zinc salt is normally 0.1 to 5 mol/L.

As the carboxylic acid used with a water-soluble zinc salt, a water-soluble carboxylic acid can be used; the examples include formic acid, acetic acid, propionic acid, oxalic acid, citric acid, tartaric acid, succinic acid, and maleic anhydride. Among them acetic acid is especially preferable.

The concentration of a carboxylic acid, in the suspension to be neutralized with an alkali, is normally 0.1 to 5 mol/L, and preferably 0.3 to 3 mol/L, though it depends on the kind of carboxylic acid, etc.. The pH of the suspension is normally 4 or less, and preferably 3.5 or less. If the amount of carboxylic acid is too small, the produced particles tend to aggregate. Even if an excess amount is used, there is no significantly improved effect and it is uneconomical.
The water-soluble zinc salt and the carboxylic acid are added so that they are within the above-described ranges in the suspension to be neutralized with an alkali. As described above, the addition can be carried out before or after the hydrolysis with heating, simultaneously or separately.

When the above-described suspension (which contains hydrolyzate of the titanium salt obtained by the hydrolysis with heating, water-soluble zinc salt, and carboxylic acid) is neutralized with an alkali, it is preferable to carry out the neutralization by the dropwise addition of the aqueous solution of an alkali.
As the alkali used in neutralization, carbonates such as sodium carbonate and potassium carbonate are suitably used, and sodium carbonate is especially preferable. At the initial stage of neutralization, other alkalis such as sodium hydroxide and potassium hydroxide can be used. However, it is preferable to use a carbonate at least from the starting point of zinc salt precipitation.

The concentration of an aqueous solution of an alkali is preferably 0.1 to 10 mol/L, and more preferably 0.1 to 2 mol/L.
In addition, the dropping rate of an aqueous solution of an alkali is preferably 0.1 to 10 mL/min for 100 mL of the zinc salt aqueous solution, and more preferably 0.1 to 5 mL/min.
If the concentration of an alkali or the dropping rate is too low, the reaction takes too long to be efficient. On the other hand, if the concentration of an alkali or the dropping rate is too high, the performance of the products may vary because the alkali is nonuniformly present in the system.

When an aqueous solution of an alkali is dropwise added, a zinc salt starts to precipitate at a certain added volume. When an aqueous solution containing zinc chloride and acetic acid is neutralized with sodium carbonate aqueous solution, the pH of reaction solution at the precipitation starting point is normally 5.5 to 6.5. The pH increases up to the precipitation starting point with an increase in the added volume. After the start of precipitation, however, the alkali is consumed for the precipitation of the zinc salt. Therefore, the pH temporarily decreases even under the progress of dropwise addition, and the pH increase is very gradual after that. When the precipitation is completed, the pH increases rapidly. It is preferable to continue the dropwise addition, after the completion of the neutralization reaction, until the completion of the zinc salt precipitation. It is normally sufficient if the dropwise addition is continued until the pH of the reaction mixture reach the vicinity of 8 to 8.5.
It is preferable to carry out the neutralization reaction at 40 °C or less. If the temperature exceeds 40 °C, the functionality of the powder may not be satisfactory. If the temperature is too low, the issues such as the lowering of precipitate formation efficiency, precipitation of reaction raw materials, and freezing, are generated. Thus, the reaction temperature is normally 15 °C or higher and preferably 25 °C or higher.

Before the neutralization with an alkali, the suspension of the hydrolyzate (precipitate) obtained by the hydrolysis with heating can be heat-treated under acidic conditions. By such a heat treatment under acidic conditions, the effects such as the enlargement in the specific surface area and refinement or equalization in particle size may be achieved for porous titanium oxide particles.
It is preferable that the pH of the suspension to be heat treated under acidic conditions is 3 or less. In order to adjust the suspension to such an acidic pH, it is preferable to use acids such as sulfuric acid, nitric acid, and hydrochloric acid, and hydrochloric acid is especially preferable.
The amount of added acid is normally 1 to 8 mole equivalents with respect to the titanium in the suspension. The heating conditions are suitably determined in accordance with the used raw materials, additives, and their concentrations; they are normally in the same ranges as those for the hydrolysis with heating.

### (c) Calcination

The precipitate produced in the neutralization reaction is separated, as necessary, by the publicly known solid-liquid separation methods such as filtration and centrifugal separation. The separated solid phase is washed with water, dried, and then calcinated to convert it to zinc oxide by decarboxylation.
The calcination temperature of 350 to 450 °C is preferable. If the calcination temperature is too high, significant sintering may take place and the desired effect may not be obtained. On the other hand, if the calcination temperature is too low, the formation of zinc oxide by calcination is inefficient and it is not desirable.
The calcination time is suitably set so that it is sufficient for the formation of zinc oxide; it is normally 1 to 10 hours.

The thus obtained powder is formed by the aggregation of randomly mixed porous titanium oxide particles and zinc oxide particles.
The porous titanium oxide particles are formed by the aggregation of primary fine particles of titanium oxide, and have numerous pores (voids). The shape of the porous titanium oxide particles is not limited in particular; for example, it can be "aegagropila-like". The term "aegagropila-like" means that it is approximately spherical, and the ratio of the minor axis to the major axis is normally 0.75 or higher.
The average particle size of the porous titanium oxide particles is preferably 0.01 to 0.5 µm, and more preferably 0.03 to 0.2 µm.

On the other hand, the aggregation of primary particles, which is observed for titanium oxide particles, is hardly observed for zinc oxide particles. The shape of zinc oxide particles is not uniform, and it is not limited in particular. The average particle size of zinc oxide particles is preferably 0.02 to 0.1 µm, and more preferably 0.02 to 0.08 µm.
The aggregate powder of the present invention is formed with the above-described porous titanium oxide particles and zinc oxide particles, and the average particle size of the aggregate powder is preferably 1 µm to 300 µm, and more preferably 1 to 100 µm.

Although the aggregate powder of the present invention have large particle size compared with normal titanium oxide fine particles and zinc oxide fine particles, it displays excellent UV protection ability. The following are considered to be the causes:
(i) A dispersion state of titanium oxide particles and zinc oxide particles is formed in each aggregate of the aggregate powder because both oxide particles are mixed and aggregated therein.
(ii) The titanium oxide particle is a porous particle that is formed by the aggregation of ultra-fine primary particles; thus the apparent refractive index is low.
(iii) The zinc oxide particles are fine particles.
(iv) The aggregation of the titanium oxide particles and zinc oxide particles is loose, and there are numerous voids between them.
(v) The aggregate powder is relatively large; thus it is resistant to aggregation compared with fine particles.

Usually, when fine titanium oxide particles with UV-B protection ability are synthesized in a liquid phase through the hydrolysis with heating, a titanium salt is hydrolyzed with heating, and the obtained precipitate is generally dried at around 100 °C to obtain the product. If the calcination is carried out at a high temperature such as 400 °C, the sintering of titanium oxide particles usually occur; thus, the UV protection ability and transparency decreases significantly.
Accordingly, when the coexistence system, containing the titanium salt hydrolyzate and a basic zinc salt, is calcinated to decarboxylate the basic zinc salt, as in the method of the present invention, it is expected that the sintering of titanium oxide particles takes place and the UV-B protection ability and transparency are impaired.

In reality, however, the aggregate powder obtained by the production method of the present invention is well dispersed in the base and displays the excellent UV protection ability and transparency over a wide range from UV-B to UV-A. The reason for this is considered as follows. When the calcination is carried out, titanium oxide particles are present as porous particles, in which primary titanium oxide particles are loosely assembled, with numerous pores (voids). As a result, the sintering of the titanium oxide primary particles does not easily take place. In addition, because the zinc oxide particles are present among the porous titanium oxide particles, the sintering between the porous titanium oxide particles is also prevented.
Thus far, it has been difficult to obtain an aggregate powder of titanium oxide particles and zinc oxide particles in a liquid phase. The production method of the present invention can provide such an aggregate powder.
The aggregate powder of the present invention has relatively large particle size. Therefore, unlike fine particle powder, the powder has less dustability and is excellent in usability and handling properties.

The aggregate powder of the present invention can be suitably used in cosmetics. The blending quantity of the aggregate powder in cosmetics is suitably determined in accordance with the purpose, it is normally 0.001 mass % or higher in cosmetics, and preferably 1 mass % or higher. If the blending quantity is too small, the effect will not be obtained. The upper limit of the blending quantity is not limited in particular, and the upper limit is normally 50 mass % or less, and preferably 30 mass % or less.

For the aggregate powder of the present invention, a publicly known surface treatment may be applied as necessary. Examples include treatment with: fatty acid soaps such as aluminum stearate and zinc myristate; waxes such as candelilla wax and carnauba wax; silicones such as methyl polysiloxane and cyclic silicone oil; dextrin fatty acid esters such as dextrin palmitate; fatty acids such as myristic acid and stearic acid; and fluorinating agent such as perfluoroalkyl phosphate.
The aggregate powder of the present invention is easily dispersible without special high dispersion treatment; therefore, there is no possibility that the surface treatment agent applied on the powder is removed during dispersion.
In addition, the UV absorption properties can be expected to be changed by further coating the powder surface with other metal species.

In the cosmetics of the present invention, other components that are normally used in cosmetics can be blended. Examples include oil, moisturizers, surfactants, pigments, dyes, powder, antioxidants, preservatives, pH adjusters, chelating agents, perfumes, UV absorbers, whitening agents, water, and various drugs.
The cosmetics of the present invention can be provided in any form such as a powder form, solid form, ointment form, liquid form, emulsion form, or a solid-liquid separated form.

Examples of product forms include basic cosmetics such as lotion, milky lotion, and cream; make-up cosmetics such as foundation, pre-makeup, lipstick, eye shadow, cheek color, eye liner, nail enamel, and mascara; and hair cosmetics such as hair treatment, hair cream, hair liquid, and setting lotion. It is especially effective to blend the aggregate powder of the present invention in sunscreen cosmetics for UV protection.
In addition to cosmetics, the aggregate powder of the present invention is applicable to other UV protection applications. Examples include resin compositions, paint, ink, and coating compositions; however, the applications are not limited to these.

### EXAMPLES

### Production Example 1 Production of titanium oxide-zinc oxide aggregates powder

The powder was produced under the following conditions.

**TABLE 1**

| | |
|---|---|
| TiCl₄ aqueous solution 1mol/L | 100mL |
| Glycerin | 0.2mol |
| ZnCl₂ | 0.1mol |
| Acetic acid | 0.1mol |
| Aqueous solution of alkali | 1mol/L NaOH (up to pH3) |
| | 0.3mol/L Na₂CO₃ (from pH3 to pH8) |
| | (added rate: 1.5ml/min) |

More specifically, 0.2 mol of glycerin was added to 100 mL of 1 mol/L titanium tetrachloride aqueous solution, and the mixture was hydrolyzed with heating (90 °C, 3 hours).
The reaction mixture was filtered, and the obtained precipitate was redispersed into 100 mL of ion-exchanged water. To the obtained suspension, 0.1 mol of zinc chloride and 0.1 mol of acetic acid were added.
To this suspension, 1 mol/L sodium hydroxide aqueous solution was dropwise added to pH 3 at 25 °C; subsequently, 0.3 mol/L sodium carbonate aqueous solution was dropwise added to pH 8. The dropping rate was 1.5 mL/min in both addition.
The neutralized reaction mixture was filtered, and the obtained precipitate was washed with water, dried (105 °C, 12 hours), and calcinated (400 °C, 3 hours) to obtain the powder of "Production Example 1".

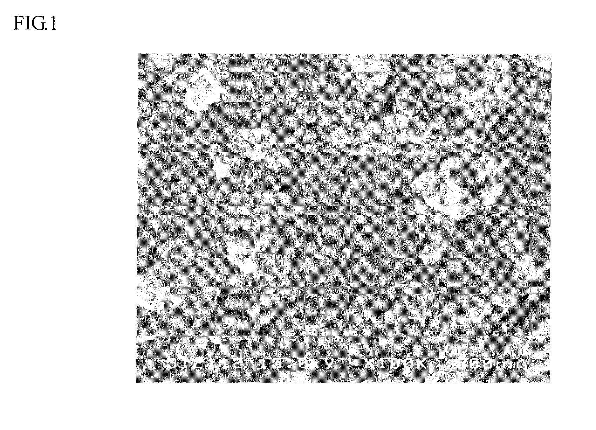

The obtained powder was, as seen in the scanning electron microscope (SEM) image in Fig. 1, aggregates of numerous particles, which were composed of particles of about 80 nm having concave-convex surfaces and particles of about 60 nm. From the observation with a transmission electron microscope, the particles of about 80 nm having concave-convex surfaces were inferred to be titanium oxide because they were porous and the particles of about 60 nm were inferred to be zinc oxide. In addition, this aggregate powder was confirmed to be composed of titanium oxide and zinc oxide by X-ray diffraction.
Based on these, the powder in "Production Example 1" was considered to be aggregates formed by the aggregation of mixed porous titanium oxide particles and zinc oxide particles.

### Test Example 1 Light Transmittance

In 3 g of castor oil, 2 g of the powder of "Production Example 1" was sufficiently ground and dispersed with a three-roll mill, the obtained dispersion was further diluted with castor oil so that the powder concentration would be 5 mass %, and the transmittance was measured at a film thickness of 5 µm.
For comparison, the same measurement was performed with the use of commercial products. "Comparative Example 1" is a 1:1 mixture (mass ratio) of commercial titanium oxide fine particles (ST485, average particle size: about 10 nm × 50 nm, manufactured by Titan Kogyo, Ltd.) and commercial zinc oxide fine particles (Finex50, average particle size: about 60 nm, manufactured by Sakai Chemical Industry Co., Ltd.).

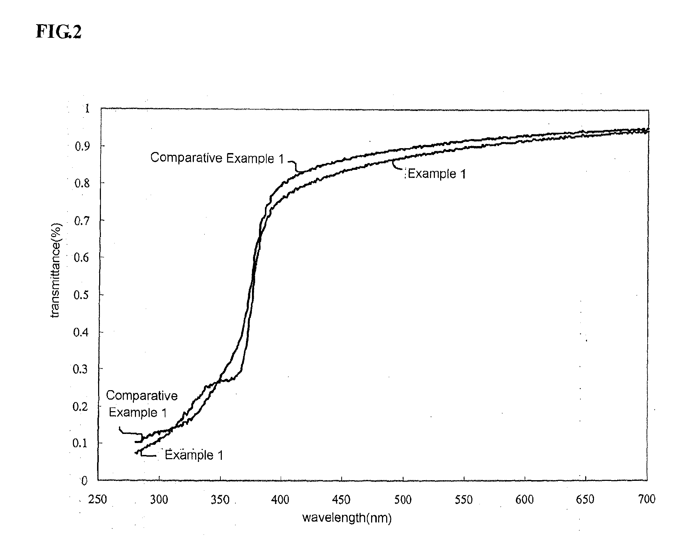

Fig. 2 shows respective transmittances in the range from 280 nm to 700 nm.
As seen from Fig. 2, the powder of "Production Example 1" displayed higher UV-B and UV-A protection ability than "Comparative Example 1" (titanium oxide fine particles : zinc oxide fine particles = 1:1).
Thus, the aggregate powder of the present invention, in a base, can protect against ultraviolet light over a wide range from UV-A to UV-B without special high dispersion treatment.

### Test Example 2 Effect of Polyhydric Alcohol

A powder was prepared in the same way as "Production Example 1" except for not using glycerin. It was observed that the primary particles were strongly-aggregated to about 1 µm in the obtained powder. When the transmittance was measured, the transparency in the visible region was low, and the protection effect in the UV-A and UV-B regions was not found. Thus, it was inferred that porous titanium oxide was not synthesized.

### Test Example 3 Effect of Acid

A powder was prepared in the same way as "Production Example 1" except for not using acetic acid. The obtained powder was aggregates in which particles of about 80 nm having concave-convex surfaces and particles of about 60 nm were mixed. However, the strong aggregation was observed among particles of about 60 nm.

### Test Example 4 Effect of Alkali

A powder was prepared in the same way as "Production Example 1" except that 0.3 mol/L sodium carbonate aqueous solution was used from the start to the end of neutralization instead of 1 mol/L NaOH aqueous solution. The obtained powder was aggregates of porous titanium oxide particles and zinc oxide particles just like that of "Production Example 1".
On the other hand, when 1 mol/L NaOH aqueous solution was used from the start to the end of neutralization, instead of 0.3 mol/L sodium carbonate aqueous solution, in "Production Example 1", the particles, which were inferred to be zinc oxide, were observed to grow large or be aggregated in the obtained powder, and the transparency was low and the protection effect in the UV-A and UV-B regions was not found.

## Claims

1. A titanium oxide-zinc oxide aggregate powder comprising an aggregate formed by the aggregation of porous titanium oxide particles and zinc oxide particles, and wherein said porous titanium oxide particle is an aggregate of primary fine particles of titanium oxide.

2. The powder according to claim 1, wherein the average particle size of the porous titanium oxide particles is 0.01 to 0.5 µm.

3. The powder according to claim 1 or 2, wherein the average particle size of the zinc oxide particles is 0.02 to 0.1 µm.

4. The powder according to any of claims 1 to 3, wherein the average particle size of the aggregate powder is 1 to 300 µm.

5. A production method of a titanium oxide-zinc oxide aggregate powder comprising the steps of:
(a) hydrolyzing, with heating, an aqueous solution containing a titanium salt and an aliphatic alcohol;
(b) adding a water-soluble zinc salt to a suspension of a precipitate obtained by the hydrolysis with heating in step (a), and then neutralizing the suspension with an alkali; and
(c) calcining a precipitate obtained by the neutralization in step(b).

6. The method according to claim 5, wherein a carboxylic acid is present in step (a) and/or step (b).

7. The method according to claim 5 or 6, wherein the precipitate obtained in step (a) is further heat-treated with an acid.

8. The method according to any of claims 5 to 7, wherein the aliphatic alcohol is a polyhydric alcohol.

9. The method according to any of claims 6 or 8, wherein the carboxylic acid is acetic acid.

10. The method according to any of claims 5 to 9, wherein the alkali is sodium carbonate.
